# EUROPÄISCHE PATENTSCHRIFT

(11) **EP 2 120 811 B1**
(45) Veröffentlichungstag und Bekanntmachung des Hinweises auf die Patenterteilung: **22.12.2010**
(21) Anmeldenummer: 08706857.3
(22) Anmeldetag: 31.01.2008
(51) Int. Cl.: A61F 5/01

(54) **VERFAHREN ZUR HERSTELLUNG EINER ORTHESE**
METHOD FOR PRODUCING AN ORTHESIS
PROCÉDÉ DE PRODUCTION D'UNE ORTHÈSE

(30) Priorität: 31.01.2007 DE 102007005733; 28.08.2007 DE 102007040467
(43) Veröffentlichungstag der Anmeldung: 25.11.2009
(73) Patentinhaber: Otto Bock HealthCare GmbH, 37115 Duderstadt (DE)
(72) Erfinder: OTTLEBEN, Michael, 37589 Kalefeld (DE)
(74) Vertreter: Lins, Edgar
(86) Internationale Anmeldenummer: PCT/DE2008/000192
(87) Internationale Veröffentlichungsnummer: WO 2008/092443

(56) Entgegenhaltungen:
- EP-A- 1 317 912
- US-A- 4 672 955
- US-A- 5 573 501
- US-A1- 2002 123 709
- US-A1- 2004 176 714

## Beschreibung

Die Erfindung betrifft ein Verfahren zur Herstellung einer Orthese mit wenigstens einem plattenförmigen Teil, das einem Körperteil angepasst ist.

Orthesen dienen dazu, Körperteile zu stützen bzw. zu halten, deren Funktion durch Krankheit oder Unfall beeinträchtigt ist. Dabei liegt die Orthese mit wenigstens einem plattenförmigen Teil an dem Körperteil an. Insbesondere bei dauernd oder über einen langen Zeitraum zu tragenden Orthesen ist es wichtig, dass das plattenförmige Teil an das Körperteil gut angepasst ist.

Durch die DE 195 06 912 C2 ist ein Verfahren zur Herstellung einer Kniegelenkorthese bekannt, die aus einem Ober- und einem Unterschenkelteil besteht, welche mittels Gelenke miteinander verbunden sind und individuell nach einem Gipsabdruck herstellbar sind. Eine dem Oberschenkel und eine dem Unterschenkel zugeordnete Platte aus warmverformbaren Kunststoff wird auf eine, durch das nach einem Gipsabdruck hergestellte Modell definierte, in die Plattenebene abgewickelte Form zugeschnitten. Die zugeschnittenen Platten werden auf eine vorbestimmte Temperatur erwähnt, die eine Verformung ermöglicht. Die erwärmten und zugeschnittenen Platten werden am hergestellten Modell angeformt und mit Bandagen befestigt. Auf diese Weise wird ein Ober- und ein Unterschenkelteil gebildet. An dem hergestellten Modell werden Gelenkadapter angebracht und die Gelenke werden daran befestigt. An den Gelenken vorgesehene Gelenkarme aus warmverformbarem Kunststoff werden erwärmt und durch Bandagen festgelegt. Brückenelemente in Form von Verbindungsstreifen aus warmverformbaren Kunststoff werden nach der Erwärmung am Ober- und Unterschenkelteil so angeformt, dass sie sich zwischen den Enden der Gelenkarme erstrecken. Nach dem Erkalten werden die Bandagen entfernt und die einzelnen Teile des kompletten Orthesenaufbaus nach der entsprechenden Anformung mit Kunststoffklebstoff verbunden. Dieses Verfahren ist aufwändig und erfordert die Herstellung eines Modells, auf dem dann die Orthese gefertigt wird, mittels eines Gipsabdruckes.

Ein Verfahren gemäß des Oberbegriffs von Anspruch 1 ist aus US 4,672,955 bekannt.

Der vorliegenden Erfindung liegt die Aufgabe zugrunde, ein Verfahren anzugeben, mit dem die Anpassung der plattenförmigen Teile an das Körperteil in vereinfachter Weise möglich ist.

Diese Aufgabe wird bei einem erfindungsgemäßen Verfahren dadurch gelöst, dass ein verformbarer plattenförmiger Träger mit einem aushärtbaren Material versehen wird und dass nach einer Anpassung der Form des Trägers durch Verformung unmittelbar auf dem Körperteil das auf dem Träger befindliche aushärtbare Material in der Form des Trägers ausgehärtet wird.

Das erfindungsgemäße Verfahren beruht somit darauf, dass das aushärtbare Material mit dem verformbaren plattenförmigen Träger durch Verformung des plattenförmigen Trägers unmittelbar auf dem Körperteil mit verformt wird und dass der plattenförmige Träger aufgrund seiner Formstabilität die Form beibehält, sodass das aushärtbare Material in der Form des verformten - nämlich an die Form des Körperteils angepassten Trägers - ausgehärtet wird.

Der plattenförmige Träger kann beim Aushärten als Teil der Orthese in dieser verbleiben oder nach dem Aushärten des aushärtbaren Materials von diesem entfernt werden. Für die Entfernung ist es ersichtlich erforderlich, dass das aushärtbare Material keine formschlüssige Verbindung mit dem plattenförmigen Träger, der auch durch ein Gittermaterial gebildet sein kann, eingeht. Hierfür kann es zweckmäßig sein, zwischen dem Träger und dem aushärtbaren Material ein Trennmittel oder eine Trennfolie einzubringen.

Das aushärtbare Material kann dabei auf die körperteilabgewandte Seite, auf die körperteilzugewandte Seite oder beiderseits des plattenförmigen Trägers aufgebracht werden. Dies gilt insbesondere, wenn als das aushärtbare Material mittels Harz vorgetränkte Kohlefasern, ein kunstsoffbeschichtetes Faserverbundmaterial oder ein thermoplastisches Faserverbundmaterial eingesetzt erden. Bevorzugt ist dabei die Verwendung von mit Harz vorgetränkten Kohlefasern, vorzugsweise in Form bekannter Prepreg-Gewebeschichten, mit denen eine Laminatschicht vorbestimmter Dicke durch Anordnung in einem Lagenpaket mit einer angepassten Zahl von Lagen realisiert werden kann. Das dabei verwendete Harz kann ein Thermoplast oder ein Duroplast sein. Während das duroplastische Harz nach der Vernetzung, insbesondere durch Wärme, praktisch nicht mehr verformbar ist, ermöglicht die Verwendung eines thermoplastischen Kunststoffes eine Nachmodellierung des plattenförmigen Teils der Orthese durch lokalen Wärmeeintrag, wie er in bekannter Weise durch Warmluft, Ultraschall, Hochfrequenz, Infrarotstrahlung usw. realisiert werden kann.

In einer anderen Ausführungsform enthält das aushärtbare Material ein Fasermaterial, das mit einer Folie oder einem Schlauch umhüllt wird, woraufhin ein Gießharz in dem vom Schlauch oder der Folie umhüllten Raum eingefüllt wird. Nach der Formgebung des plattenförmigen Trägers wird dann das Gießharz mit dem Fasermaterial als Laminat ausgehärtet. Die Aushärtung kann durch eine chemische Reaktion, durch Einbringen von Wärme usw. erfolgen. In diesen Fällen ist es daher zweckmäßig, wenn auf die körperteilzugewandte Seite des plattenförmigen Trägers ein wärmeisolierendes Material aufgebracht wird. In diesem Fall kann die Aushärtung unmittelbar nach der Verformung des plattenförmigen Trägers auf dem Körperteil selbst erfolgen. Das wärmeisolierende Material kann nach dem Aushärten des aushärtbaren Materials vorzugsweise wieder entfernt werden. An dessen Stelle kann dann ein Polster aufgebracht werden, das den Trägekomfort für die Orthese erhöht.

Das Einfüllen des Gießharzes in den mit der Folie oder dem Schlauch umhüllten Raum kann dadurch erleichtert werden, dass in dem Raum ein Unterdruck erzeugt wird. Insbesondere kann dadurch ein Einschluss von Luftblasen verhindert werden. Alternativ kann die Folie oder der Schlauch vor dem Aushärten des Gießharzes an die laminierte Platte angepresst werden.

Als Fasermaterial kommen lose, gewebte oder gewirkte oder zu einem Vlies verbundene Fasern insbesondere aus Kohlenstoff, Glas, Polyamid, Kevlar und anderen Kunststoffen in Frage.

Als Schlauch kann vorzugsweise ein Schrumpfschlauch verwendet werden.

Benötigt die Orthese ein Gelenk, wird dieses vorzugsweise durch ein externes Gestellt fixiert und dann mit den hergestellten plattenförmigen Teilen verbunden.

Der erfindungsgemäße plattenförmige Träger muss sich am Körperteil leicht verformen lassen, andererseits die dabei angenommene Form stabil beibehalten, sodass das aushärtbare Material, insbesondere das faserverstärkte Material, die Form des plattenförmigen Trägers annimmt und während des Aushärtens beibehält. Der plattenförmige Träger kann durch eine dünne Leichtmetallplatte, beispielsweise Aluminiumplatte, gebildet sein. Möglich ist ferner die Verwendung einer Gitterstruktur, die vorzugsweise aus Metall gebildet ist.

Der plattenförmige Träger kann ferner durch wenigstens zwei Lagen mit zueinander zeigenden Oberflächentopographien gebildet sein, die, beispielsweise unter Vakuum, aneinander gepresst werden und sich nach einer Verformung mit ihren Oberflächenprofilen miteinander verhaken und so die an das Körperteil angepasste Form beibehalten. Geeignete Oberflächentopographien sind Riffelprofile, aber auch unregelmäßige Profile, wie sie beispielsweise bei grobkörnigem Schleifpapier auftreten.

Als plattenförmiger Träger können ferner Bandagen verwendet werden, die durch ein Bindemittel mit Formstabilität verformbar ausgebildet sind. Das Laminiermaterial kann dabei - ggf. unter Zwischenlegen eines Polsters - auf das Körperteil aufgelegt werden und mittels der Bandage gegen den Körper gedrückt werden, sodass die Form des Körperteils von dem Laminiermaterial angenommen wird. Die Bandagen können dann mit ihrem Bindemittel verfestigt werden, wobei das Bindemittel ein sich verfestigender Kunststoff oder ein trocknender Gips sein kann. Anschließend kann das Laminiermaterial in der durch die Bandagen fixierten Formgebung ausgehärtet werden, sodass die Anpassung an die Form des Körperteils erfolgt ist.

In der beigefügten Zeichnung ist schematisch eine Ausführungsform einer erfindungsgemäß hergestellten Orthese dargestellt.

Es zeigen:
- Figur 1: einen schematischen Aufbau der Schichten einer erfindungsge- mäßen Orthese und
- Figur 2: einen Schnitt durch ein Körperteil, beispielsweise Oberschenkel, mit angelegter Orthese gemäß Figur 1.

In den Figuren der Zeichnung sind gleiche und einander entsprechende Bauteile mit gleichen Bezugszeichen versehen.

Die Zeichnung zeigt schematisch eine Orthese 2, die mit einem plattenförmigen Teil an ein Körperteil 3, beispielsweise einen Oberschenkel, hinsichtlich Größe und Form anpassbar ist.

Die Orthese 2 weist einen plattenförmigen Träger 4, hier in Form einer dünnen Aluminiumplatte, auf, auf dessen körperteilabgewandten Seite ein Material bzw. Stoff, beispielsweise Kohlefasern o. ä., auflaminiert wird. Hierzu wird auf die Platte 4 Fasermaterial 5, beispielsweise eine Kohlefaserschicht, drapiert und mit einem Harz, beispielsweise Epoxidharz, getränkt. Hierfür wird zunächst die Platte 4 mit dem aufgebrachten Fasermaterial 5 mit einem Schlauch oder einer Folie 6 dicht umzogen, deren Rand die Platte 4 untergreift und mit der Platte 4 verbunden wird, beispielsweise mittels eines Klebers 17 verklebt wird. In den von der Folie 6 umhüllten Raum 8 wird dann Gießharz 9 eingefüllt, beispielsweise mittels einer Spritze eingespritzt, dass das Fasermaterial 5 durchtränkt. Die so behandelte Platte 4 wird dann an das Körperteil 3 zur Formung der Orthese angepasst. Danach wird das Gießharz 9 ausgehärtet und nimmt dabei die Form der verformten Platte 4 an.

Das Laminieren und das Einfüllen des Gießharzes kann dabei bei noch nicht angelegter und noch nicht angepasster Orthese bereits erfolgen.

Das Laminieren mit Gießharz und Kohlefasern kann auch direkt auf das Körperteil 3 erfolgen, wobei allerdings nach dem Aufbringen des zu laminierenden Materials 5 auf die körperteilabgewandte Seite der Platte 4 oder auf beide Seiten der Platten 4 zunächst ein wärmeisolierendes Material auf die körperteilzugewandte Seite der Platte 4 oder auf beide Seiten der Platten 4 aufgebracht wird, um zu verhindern, dass bei einer chemischen Reaktion beim Aushärten des Gießharzes entstehende Hitze auf die Haut des Körperteils 3 einwirkt.

Danach wird wie bei dem zuerst beschriebenen Verfahren die soweit vorbereitete Platte 4 mit einer Folie 6 umzogen und es wird dann in den von der Folie 6 umhüllten Raum 8 Gießharz 9 eingefüllt. Danach erfolgt das Anpassen der soweit vorbereiteten und noch formbaren Platte 4 an das Körperteil 3 zur Formung der Orthese. Danach härtet das Gießharz 9 aus und es wird auf die körperteilzugewandte Seite der Orthese ein Polster 10 aufgebracht, wobei vorzugsweise zuvor das wärmeisolierende Material entfernt wird.

Es besteht auch die Möglichkeit, nach dem Aufbringen von zu laminierendem Material, d. h. von Fasermaterial 5, auf die körperteilabgewandte Seite der Platte 4 und nach dem Umziehen der soweit behandelten Platte 4 mit der Folie 6 zunächst auf die körperteilzugewandte Seite der Platte 4 ein Polster 10 aufzubringen und dann die soweit vorbereitete Platte 4 an das Körperteil 3 zur Formung der Orthese anzupassen. Danach wird die an das Körperteil 3 angeformte Orthese 2 entfernt und es wird in den von der Folie 6 umhüllten Raum 8 Gießharz 9 zum Durchtränken des Fasermaterials eingefüllt und zum Aushärten gebracht.

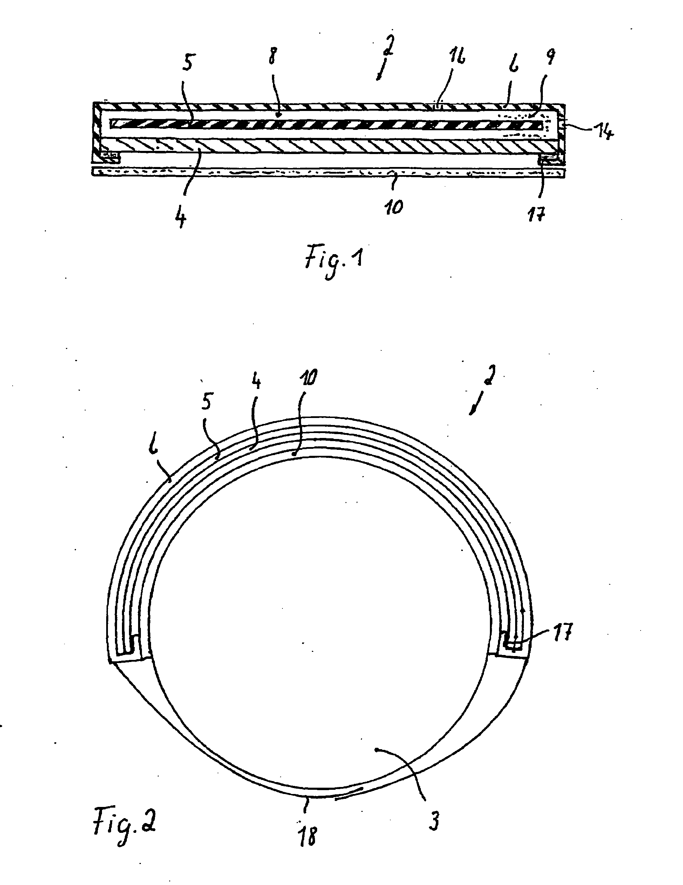

Durch Anpressen der Folie 6 an die laminierte Platte 4 vor dem Aushärten des Gießharzes 9 kann der Raum 8 entlüftet werden, um so ein gleichmäßiges Laminat herzustellen. Dies gelingt noch besser, wenn in dem mit dem Gießharz 9 gefüllten Raum 8 ein Unterdruck erzeugt wird, durch den der Verbund aus Folie 6, Laminat und Platte 4 zusammengepresst wird, wodurch eventuell vorhandener Gießharzüberschuss und Luft vor dem Aushärten des Gießharzes 9 abgezogen wird. Dieser Unterdruck kann durch eine Pumpe erzeugt werden, die an eine geeignete Öffnung 14 (gestrichelt in Figur 1 eingezeichnet) der Folienumhüllung 6 angeschlossen wird. Über eine zweite, am oberen Teil angebrachte Öffnung 16 (gestrichelt in Figur 1 eingezeichnet) kann dann überschüssiges Gießharz 9 und Luft nach oben hin abfließen. Anstelle der beschriebenen umgelegten Folie 6 und der Pumpe zur Erzeugung eines Unterdrucks kann auch ein Schrumpfschlauch eingesetzt werden.

Der Anpressdruck kann alternativ mit Hilfe elastischer Magnetplatten (nicht dargestellt) erfolgen.

Bei Vorhandensein eines Gelenks, beispielsweise eines Kniegelenks, wird dieses durch ein externes Gestell (nicht dargestellt) fixiert.

Für besonders feste Orthesen empfiehlt es sich, einen Kern (nicht dargestellt) in die Mitte des zu laminierenden Stoffes 5 einzubringen, wobei dieser Kern auch so gewählt werden kann, dass er die Funktion der Platte 4 durch leichte und formstabile Verformbarkeit übernehmen kann.

Auf die körperteilzugewandte Seite der Orthese 2 kann vor oder nach dem Aushärten des Gießharzes das Polster 10 aufgebracht werden.

An der Stelle des Fasermaterials 5, das mit dem Gießharz 9 durchtränkt wird, kann auch bereits ein mit einem Harz, beispielsweise Epoxidharz, vorgetränktes Fasernaterial, beispielsweise eine mit Harz bzw. Epoxidharz vorgetränkte Kohlefaserschicht 5, eingesetzt werden. Es entfällt dann das Einfüllen von Gießharz 9 in den von der Folie 6 umhüllten Raum 8. Wenn ein vorgetränktes Fasermaterial verwendet wird, wird auch die Folie 6 nicht mehr benötigt.

Das zu laminierende Material 5 kann auch auf die körperteilzugewandte Seite der Platte 4 oder auf beide Seiten der Platten 4 aufgebracht werden. Das Laminieren mit dem mit Harz vorgetränkten Material bzw. mit Harz vorgetränkter Kohlefaserschicht kann direkt auf dem Körperteil 3 erfolgen.

Figur 2 zeigt schematisch einen Schnitt durch einen Körperteil 3, beispielsweise einen Oberschenkel, mit einer erfindungsgemäßen Orthese 2 in Form einer plattenförmigen Schale, bestehend aus einer außen oder beidseitig mittels Kohlefasern 5 laminierten Aluminiumschale 4, auf deren Innenseite (körperteilzugewandte Seite) ein Polstermaterial 10 angeordnet ist und die von einer nachgiebigen Folie 6 überlagert ist, deren Rand die Aluminiumschale 4 untergreift und mit der Aluminiumschale 4 verbunden ist, beispielsweise verklebt (Kleber 17) ist. In dem von der Folie 6 eingehüllten Raum 8 befindet sich Gießharz 9, das im ausgehärteten Zustand die Formstabilität der Orthese 2 ergibt. Mittels eines Verschlusselementes 18, beispielsweise eines Klettverschlusses, kann die Orthese 2 am Körperteil 3, beispielsweise im Oberschenkel, angebracht werden.

Der Aufbau der im Übrigen gleich ausgebildeten Orthese 2 ändert sich durch die Verwendung von mit Harz vorgetränkten Kohlefasern 5 dadurch, dass keine Folie 6 mehr benötigt wird.

Als zu laminierendes Material oder anstelle von mit Harz vorgetränktem Material kann auch kunststoffbeschichtetes Faserverbundmaterial oder thermoplastisches Faserverbundmaterial eingesetzt werden. Das Aushärten erfolgt vorzugsweise unter Zufuhr von Wärme.

## Patentansprüche

1. Verfahren zur Herstellung einer Orthese (2) mit wenigstens einem plattenförmigen Teil, das einem Körperteil (3) angepasst ist, wobei ein verformbarer plattenförmiger Träger (4) mit einem aushärtbarem Material (5, 9) versehen wird und dass nach einer Anpassung der Form des Trägers (4) durch Verformung unmittelbar auf dem Körperteil (3) das auf dem Träger (4) befindliche aushärtbare Material (5,9) in der Form des Trägers (4) ausgehärtet wird, **dadurch gekennzeichnet, dass** der Träger (4) die angepasste Form aufgrund seiner Formstabilität beibehält.

2. Verfahren nach Anspruch 1, **dadurch gekennzeichnet, dass** der plattenförmige Träger (4) als Teil der Orthese (2) in dieser verbleibt.

3. Verfahren nach Anspruch 1, **dadurch gekennzeichnet, dass** der plattenförmige Träger (4) nach dem Aushärten des aushärtbaren Materials (5, 9) von diesem entfernt wird.

4. Verfahren nach einem der Ansprüche 1 bis 3, **dadurch gekennzeichnet, dass** das aushärtbare Material Kohlefasern enthält oder eine mit Harz vorgetränkte Kohlefaserschicht ist.

5. Verfahren nach Anspruch 4, **dadurch gekennzeichnet, dass** das zum Vortränken der Kohlefaserschicht verwendete Harz ein Thermoplast ist.

6. Verfahren nach Anspruch 5, **dadurch gekennzeichnet, dass** das ausgehärtete Material durch lokalen Wärmeeintrag lokal verformt wird.

7. Verfahren nach einem der Ansprüche 1 bis 6, **dadurch gekennzeichnet, dass** das aushärtbare Material (5, 9) ein Fasermaterial ist, das mit einer Folie (6) oder einem Schlauch umhüllt wird und dass ein Gießharz (9) in dem vom Schlauch oder der Folie (6) umhüllten Raum (8) eingefüllt wird.

8. Verfahren nach einem der Ansprüche 1 bis 7, **dadurch gekennzeichnet, dass** auf die körperteilzugewandte Seite des plattenförmigen Trägers (4) ein wärmeisolierendes Material aufgebracht wird.

9. Verfahren nach Anspruch 8, **dadurch gekennzeichnet, dass** auf die körperteilzugewandte Seite des plattenförmigen Trägers ein Polster aufgebracht wird und/oder dass nach Aushärtung des Gießharzes das wärmeisolierende Material entfernt und durch ein Polster ersetzt wird.

10. Verfahren nach einem der Ansprüche 4 bis 7, **dadurch gekennzeichnet, dass** in dem mit Gießharz (9) gefüllten Raum (8) ein Unterdruck erzeugt wird.

11. Verfahren nach einem der Ansprüche 5 bis 9, **dadurch gekennzeichnet, dass** nach Aushärtung des Gießharzes (9) das wärmeisolierende Material entfernt und durch ein Polster (10) ersetzt wird.

12. Verfahren nach einem der Ansprüche 1 bis 10, **dadurch gekennzeichnet, dass** der plattenförmige Träger (4) durch eine dünne Leichtmetallplatte gebildet ist.

13. Verfahren nach einem der Ansprüche 1 bis 10, **dadurch gekennzeichnet, dass** der plattenförmige Träger (4) durch eine Gitterstruktur, vorzugsweise aus Metall, gebildet ist.

14. Verfahren nach einem der Ansprüche 1 bis 12, **dadurch gekennzeichnet, dass** der plattenförmige Träger (4) aus wenigstens zwei Schichten besteht, die mit jeweils einer miteinander verhakenden Oberflächenstruktur aneinander anliegen.

15. Verfahren nach einem der Ansprüche 1 bis 10, **dadurch gekennzeichnet, dass** der plattenförmige Träger (4) durch Bandagen gebildet ist, die durch ein Bindemittel mit Formstabilität verformbar ausgebildet sind.

## Claims

1. Method for producing an orthosis (2) with at least one plate-shaped part which is adapted to a body part (3), a deformable plate-shaped support (4) being provided with a curable material (5, 9) and, after the shape of the support (4) has been adapted by deformation directly on the body part (3), the curable material (5, 9) present on the support (4) being cured in the shape of the support (4), **characterized in that** the support (4) retains the adapted shape by virtue of its dimensional stability.

2. Method according to Claim 1, **characterized in that** the plate-shaped support (4) remains in the orthosis (2) as part thereof.

3. Method according to Claim 1, **characterized in that** the plate-shaped support (4) is removed from the curable material (5, 9) after the latter has been cured.

4. Method according to one of Claims 1 to 3, **characterized in that** the curable material contains carbon fibers or is a carbon fiber layer pre-impregnated with resin.

5. Method according to Claim 4, **characterized in that** the resin used to pre-impregnate the carbon fiber layer is a thermoplastic.

6. Method according to Claim 5, **characterized in that** the cured material is deformed locally by local application of heat.

7. Method according to one of Claims 1 to 6, **characterized in that** the curable material (5, 9) is a fiber material enclosed by a film (6) or a tube, and **in that** a casting resin (9) is introduced into the space (8) enclosed by the tube or the film (6).

8. Method according to one of Claims 1 to 7, **characterized in that** a heat-insulating material is applied on the side of the plate-shaped support (4) directed toward the body part.

9. Method according to Claim 8, **characterized in that** a pad is applied on the side of the plate-shaped support directed toward the body part and/or **in that**, after the casting resin has been cured, the heat-insulating material is removed and replaced by a pad.

10. Method according to one of Claims 4 to 7, **characterized in that** an underpressure is generated in the space (8) filled with casting resin (9).

11. Method according to one of Claims 5 to 9, **characterized in that**, after the casting resin (9) has been cured, the heat-insulating material is removed and replaced by a pad (10).

12. Method according to one of Claims 1 to 10, **characterized in that** the plate-shaped support (4) is formed by a thin plate of light metal.

13. Method according to one of Claims 1 to 10, **characterized in that** the plate-shaped support (4) is formed by a lattice structure, preferably of metal.

14. Method according to one of Claims 1 to 12, **characterized in that** the plate-shaped support (4) is composed of at least two layers which bear on each other by means of their respective surface structures hooking onto each other.

15. Method according to one of Claims 1 to 10, **characterized in that** the plate-shaped support (4) is formed by bandages that are made deformable by a binder with dimensional stability.

## Revendications

1. Procédé pour la fabrication d'une orthèse (2) avec au moins une partie en forme de plaque, qui s'adapte à un élément corporel (3), dans lequel une plaque porteuse (4) déformable de plaque est munie d'un matériau durcissable (5, 9) et dans lequel le matériau durcissable (5, 9) se trouvant sur la plaque porteuse (4) est durci à la forme de la plaque porteuse (4) par une adaptation de la forme de la plaque porteuse (4) par déformation directe sur l'élément corporel (3), **caractérisé en ce que** la plaque porteuse (4) conserve la forme adaptée du fait de sa résistance de forme.

2. Procédé selon la revendication 1, **caractérisé en ce que** la plaque porteuse (4) reste dans l'orthèse (2) en tant que partie de celle-ci.

3. Procédé selon la revendication 1, **caractérisé en ce que** la plaque porteuse (4) est séparée du matériau durcissable (5, 9) après durcissement de celui-ci.

4. Procédé selon l'une quelconque des revendications 1 à 3, **caractérisé en ce que** le matériau durcissable contient des fibres de carbone ou une couche de fibres de carbone préimprégnée de résine.

5. Procédé selon la revendication 4, **caractérisé en ce que** l'âme utilisée pour la résine utilisée pour la préimprégnation de la couche de fibres de carbone est un thermoplastique.

6. Procédé selon la revendication 5, **caractérisé en ce que** le matériau durci est déformé localement par apport local de chaleur.

7. Procédé selon l'une des revendications 1 à 6, **caractérisé en ce que** le matériau durcissable (5, 9) est un matériau à base de fibre, qui est entouré par une feuille (6) ou un tube et **en ce qu'**une résine de coulée (9) est injectée dans l'espace (8) entouré par le tube ou la feuille (6).

8. Procédé selon l'une des revendications 1 à 7, **caractérisé en ce qu'**un matériau thermiquement isolant est prévu sur le côté de la plaque porteuse (4) tournée vers l'élément corporel.

9. Procédé selon la revendication 8, **caractérisé en ce qu'**un rembourrage est prévu sur le côté de la plaque porteuse tournée vers l'élément corporel, et/ou **en ce que** le matériau thermiquement isolant est retiré et remplacé par un rembourrage après durcissement de la résine de coulée.

10. Procédé selon l'une des revendications 4 à 7, **caractérisé en ce que** une pression est appliquée à l'espace (8) rempli par la résine de coulée (9).

11. Procédé selon l'une des revendications 5 à 9, **caractérisé en ce que** le matériau thermiquement isolant est retiré et remplacé par un rembourrage (10) après durcissement de la résine de coulée (9).

12. Procédé selon l'une des revendications 1 à 10, **caractérisé en ce que** la plaque porteuse (4) est réalisée en une mince plaque de métal léger.

13. Procédé selon l'une des revendications 1 à 10, **caractérisé en ce que** la plaque porteuse (4) est réalisée en une structure ajourée, de préférence en métal.

14. Procédé selon l'une des revendications 1 à 12, **caractérisé en ce que** la plaque porteuse (4) est constituée d'au moins deux couches, qui sont disposées l'une contre l'autre avec toutes deux une structure de surface se verrouillant l'une avec l'autre.

15. Procédé selon l'une des revendications 1 à 10, **caractérisé en ce que** la plaque porteuse (4) est réalisée par des bandages qui sont mis en oeuvre de façon déformable à l'aide d'un mortier rigide.
